# EUROPEAN PATENT APPLICATION

(11) **EP 2 657 819 A1**
(43) Date of publication of application: **30.10.2013**
(21) Application number: 11850376.2
(22) Date of filing: 24.12.2011
(51) Int. Cl.: G06F 3/048

(54) **METHOD AND DEVICE FOR EDITING ORGANIC CHEMISTRY STRUCTURAL FORMULA**

(30) Priority: 24.12.2010 CN 201010622006
(71) Applicant: Peking University Founder Group Co., Ltd, Haidian District Beijing 100871 (CN); Beijing Founder Electronics Co., Ltd., Haidian District, Beijing 100085 (CN); Peking University Founder R&D Center, Beijing 100871 (CN)
(72) Inventor: ZHAO, Zhigang, Beijing 100085 (CN)
(74) Representative: Harrison Goddard Foote LLP
(86) International application number: PCT/CN2011/084600
(87) International publication number: WO 2012/083888

(57) **Abstract**

The application provides a method for editing an organic chemical structure formal comprising a step of monitoring an input from a keyboard, a step of determining a focus at the organic chemical structure formal in an interactive chemical typesetting interface, and a step of prompting executable operations for the determined focus in the interactive chemical typesetting interface in response to the monitored input. The application further provides an apparatus for editing an organic chemical structure formal. The apparatus may comprise a monitoring module configured to monitor an input from a keyboard, a focus module configured to determine a focus at the organic chemical structure formal in an interactive chemical typesetting interface, and a showing module configured to show executable operations for the determined focus in the interactive chemical typesetting interface in response to the monitored input. According to the present application, operation efficiency of editing organic chemical structure formals may be improved.

## Description

### Technical Field

The present application relates to a field of digital typesetting, in particular, to a method and an apparatus for editing an organic chemical structure formula.

### Background

In the existing interactive chemical typesetting software, the organic chemical structure formals are usually typeset through a number of controls in a graph interface. A user is required to take various controls in the graph interface by operating the mouse to edit (such as construct/create or modify) the organic chemical structure formals. It seems to be intuitive, but it is actually difficult to grasp the method fast and it is more difficult to improve the operating speed.

### Summary

The present application intends to provide a method and an apparatus for editing an organic chemical structure formula so as to at least simplify complicated operations for inputting organic chemical structure formulas in the prior art.

According embodiments of the present application, a method for editing an organic chemical structure formula is provided. The method may comprise a step of monitoring an input from a keyboard, a step of determining a focus at the organic chemical structure formal in an interactive chemical typesetting interface, and a step of prompting executable operations for the determined focus in the interactive chemical typesetting interface in response to the input.
According embodiments of the present application, an apparatus for editing an organic chemical structure formal is provided. The apparatus comprises a monitoring module, a focus module and a prompting module. The monitoring module is configured to monitor an input from a keyboard. The focus module is configured to determine a focus at the organic chemical structure formal in an interactive chemical typesetting interface. The prompting module is configured to prompt executable operations for the determined focus in the interactive chemical typesetting interface in response to the input.

According to a method and an apparatus for editing an organic chemical structure formal provided in embodiments of the present application, complicated operations of editing organic chemical structure formals in the prior art may be simplified and operation efficiency of editing organic chemical structure formals may be improved by prompting executable operations in response to the input from the keyboard.

### Brief Description of the Drawing

The drawings described herein are used to provide a further understanding to the present application and constitute a part of this specification. Exemplary embodiments of the present application and their descriptions serve to explain the present application and do not constitute improper limitation on the present application. In the drawings:

Fig. 1 is a flowchart illustrating a method for editing an organic chemical structure formula according to an embodiment of the present application.

Fig. 2 illustrates a screenshot of prompt state of a selected chemical bond (without operations) according to a preferred embodiment of the present application.

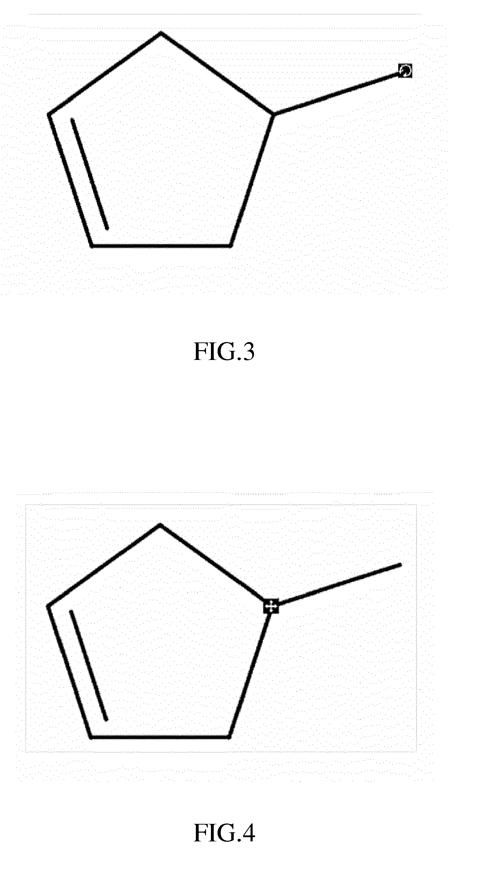

Fig. 3 illustrates a screenshot of rotating prompt state of a selected atom according to a preferred embodiment of the present application.

Fig. 4 illustrates a screenshot of moving prompt state of a selected atom according to a preferred embodiment of the present application.

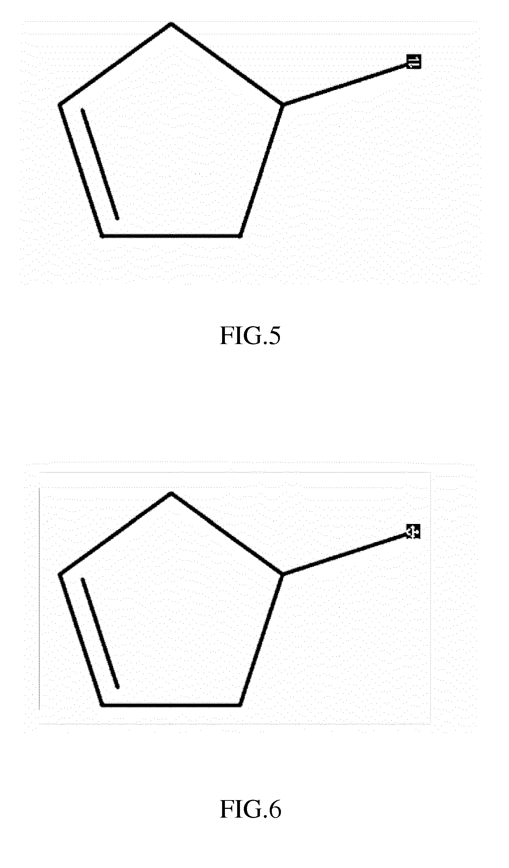

Fig. 5 illustrates a screenshot of chemical stretching prompt state of a selected atom according to a preferred embodiment of the present application.

Fig. 6 illustrates a screenshot of constructing new chemical bond prompt state of a selected atom according to a preferred embodiment of the present application.

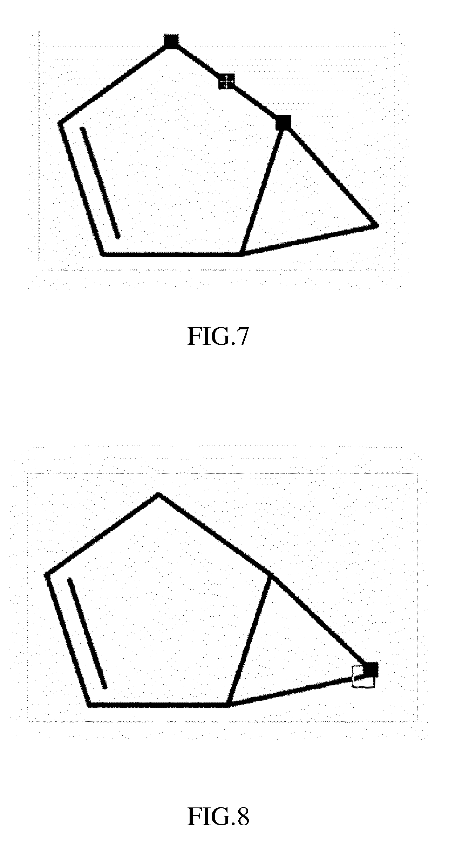

Fig. 7 illustrates a screenshot of switching key type prompt state of a selected chemical bond according to a preferred embodiment of the present application.

Fig. 8 illustrates a screenshot of merging prompt state of a selected atom merged with other atoms according to a preferred embodiment of the present application.

Fig. 9 is a schematic diagram illustrating an apparatus for editing an organic chemical structure formula according to an embodiment of the present application

### Detailed Description

Hereinafter, the present application will be explained in detail with reference to the accompanying drawings in connection with embodiments thereof.

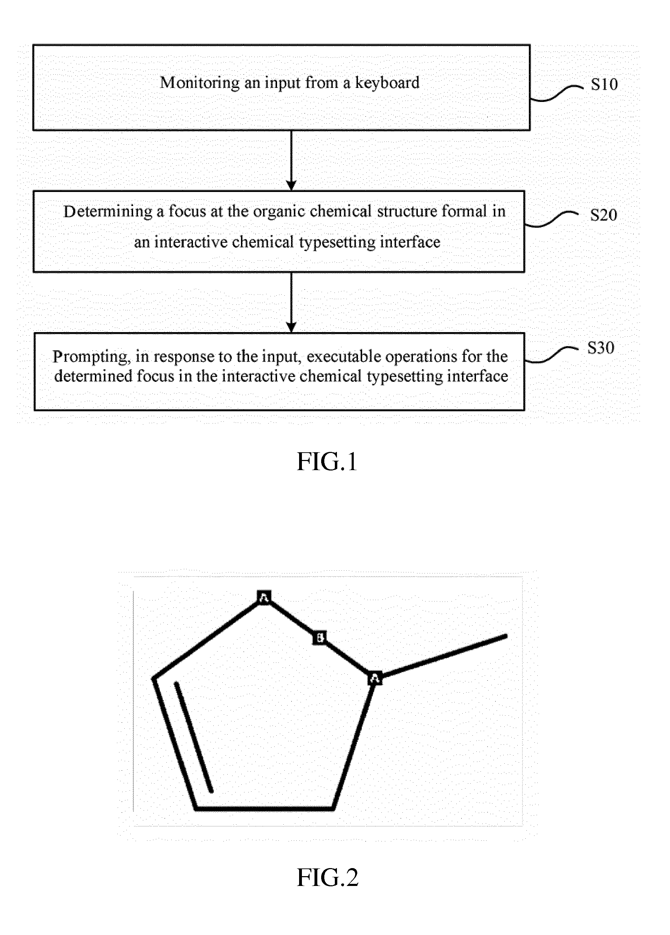

Fig. 1 is a flowchart illustrating a method for editing an organic chemical structure formula according to an embodiment of the present application, the method may comprise:
a step S10, in which it monitors an input from a keyboard;
a step S20, in which a focus at the organic chemical structure formal is determined in an interactive chemical typesetting interface; and
a step S30, in which executable operations for the determined focus is shown in the interactive chemical typesetting interface in response to the input.

In the existing interactive chemical typesetting software, the chemical bonds are usually edited by operations of a mouse, in which the consecutive switching operations and editing operations cannot be performed simultaneously. While, in the embodiments of the present application, the executable operations are prompted in a screen in response to the input from the keyboard, so as to facilitate the execution of corresponding keyboard operations. For example, these operations to be prompted comprise selecting, rotating, stretching, constructing, moving, merging, changing types, showing the hidden and so on. Then, the chemical bonds may be edited by a computer in response to the input from the keyboard. Compared with mouse operations, the keyboard operations may be more rapidly, directly and consecutively done, which are irreplaceable by mouse operations. Therefore, in the interactive chemical typesetting software, efficiency of users can be improved by operations of keyboard (the mouse operations for editing chemical bonds are not needed) to facilitate the user in obtaining various expected structures, so that usability and efficiency of the chemical typesetting can be improved.

The interactive operations of organic chemical structure formals may be performed depending on the selected focuses. Different types of focuses correspond to different operations, but correspond to a same button in the keyboard. Various operation prompts are provided by determining the types of the selected focus and different control keys so that the user can know clearly whether the current operation is expected or not. According to the present method, the user can understand types and ability of the current operation to prevent erroneous operation, and thus the speed and efficiency of constructing organic chemical structure formals may be improved.

For the selecting operation, when a chemical bond is selected, atoms at two ends of the bond may be selected at the same time, since a chemical bond is generated by the interaction between two atoms. However, the selection of single atom has no impact on the connected chemical bonds. Therefore, it is useful for the user to show the types of the selected contents. Fig. 2 shows that the types of the selected contents are chemical bond (*Bond* is represented as *B*) and two atoms (*Atom* is represented as *A*).

When a chemical bond is selected, there are a number of executable operations such as rotating around one end thereof, stretching, moving, or constructing a new chemical bond or an atom based on the chemical bond itself. In the case that a single atom is selected, functions corresponding to the different operation keys having been pushed down should be determined and then prompts of these functions are shown.

For example, when a single atom is selected, if the key "Ctrl" is pushed down, then it will show to the user that a new chemical bond may be constructed by cooperation of this key with direction keys.

When a single chemical bond is selected, if the key "Ctrl" is pushed down, then it will prompt that the type of the chemical bond may be switched by cooperation of this key with the direction keys.

When there are a plurality of chemical bonds connected with the selected atom, if the key "shift" is pushed down, it will show that, if the key "shift" cooperates with the direction key, the atom may be moved along the direction corresponding to the direction key, so that the plurality of chemical bonds are moved correspondingly.

When there is only one chemical bond connected with the selected atom, if the key "shift" is pushed down, it will show that, if the key "shift" cooperates with the direction keys, the chemical bond may be rotated around the other end thereof; and if the key "Alt" is pushed down, it will show that, if the key "Alt" cooperates with the direction keys, the chemical bond may be stretched around the other end thereof.

When one or more of atoms are overlapped during moving, a prompt block prompting available merging is shown in the position of the overlapped atoms.

Preferably, when it is determined that the determined focus is an atom, if the monitored input is from a first key or a first key combination, the shown executable operations in step S30 may indicate that: the first key or key combination together with a specific key is capable of constructing a bond connected to the atom. For example, it will show the key "Ctrl" plus the direction keys, which indicates that a new chemical bond may be constructed for a selected atom.

Preferably, when it is determined that the focus is an atom, if the monitored input is a second key or a second key combination and if one bond is connected with the atom, the shown executable operations in step S30 may indicate that: the second key or key combination together with a specific key is capable of rotating the bond around the atom. For example, when the key "shift" is pushed, it will prompt that the operation refers to rotating. If the monitored input is from the second key or the key combination and a plurality of bonds are connected with the atom, the shown executable operations in step S30 may indicate that: the second key or key combination together with a specific key is capable of moving the atom and changing the plurality of bonds accordingly. For example, it will prompt that the pushed key "shift" together with the direction keys is used to carry out the function of moving.

Preferably, when it is determined that the focus is a bond, if the monitored input is a first key or a first key combination, the shown executable operations in step S30 may indicate that: the first key or key combination together with the specific keys is capable of switching the type of the bond. For example, it will prompt that: "Ctrl" and the direction keys are capable of switching the types of the bond.

In the above-mentioned preferable embodiments, for a selected atom, "Ctrl" and the direction keys are used to construct a chemical bond, while for a selected chemical bond, they are used to switch types of chemical bonds. Various types of chemical bonds comprise a fixed bond, a double bond, an upward stereoscopic bond and downward stereoscopic bond, in which the fixed bond comprises the following types such as a single bond, a triple bond, a trans-double bond and a quadruple bond. Prompt states of the focus are determined according to the above conditions and the corresponding operation prompts are then provided. It can be seen that, with the prompts, the operation failures and then learning burden can be reduced.

Preferably, different operations may be shown in different colors to facilitate the user's selection. Although all the figures herein are shown in black and white, the skilled in the art would understand that those figures can be achieved in colors. For example, a modifying operation may be shown in red, and a constructing operation may be shown in green. Note that, operations for focus may be prompted in real time. The prompts are shown when the corresponding buttons are pushed down until the operation is over.

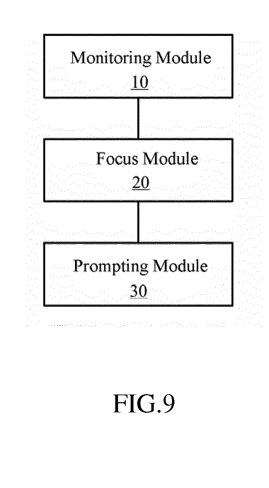

Fig. 9 is a schematic diagram illustrating an apparatus for editing an organic chemical structure formal according to an embodiment of the present application. The apparatus may comprise a monitoring module 10 configured to monitor an input from a keyboard, a focus module 20 configured to determine a focus at an organic chemical structure formal in an interactive chemical typesetting interface, and a prompting module 30 configured to prompt executable operations for the determined focus in the interactive chemical typesetting interface in response to the input.

The usability and efficiency of chemical typesetting can be improved by the apparatus.

Preferably, when it is determined that the determined focus is an atom, if the monitored input is from a first key or a first key combination, the shown executable operations may indicate that: the first key or key combination together with a specific key is capable of constructing a bond connected to the atom.

Preferably, if the monitored input is a second key or a second key combination and if one bond is connected with the atom, the shown executable operations may indicate that: the second key or key combination together with a specific key is capable of rotating the bond around the atom. If the monitored input is from the second key or the key combination and a plurality of bonds are connected with the atom, the shown executable operations may indicate that: the second key or key combination together with a specific key is capable of moving the atom, and changing the plurality of bonds accordingly.

Preferably, when it is determined that the focus is a bond, if the monitored input is a first key or a first key combination, the shown executable operations may indicate that: the first key or key combination together with the specific keys is capable of switching the type of the bond.

Preferably, different operations may be shown in different colors.

From the above, according to the above embodiments of the present invention, various operation prompts are provided by determining the types of the selected focus and different control keys so that the user can know clearly whether the current operation is expected or not. According to the present application, the user can understand types and ability of the current operation to prevent erroneous operation so as to improve the speed and efficiency of constructing organic chemical structure formals. Accordingly, the efficiency of typesetting may be improved and learning difficulty may be reduced.

It will be readily apparent to those skilled in the art that respective modules or steps of the present application may be implemented with a common computing device. In addition, the modules or steps of the present application may be concentrated in a single computing device or distributed in a network composed of multiple computing devices. Optionally, the modules or steps may be achieved by using codes of executable programs, so that they can be stored in a storage medium, or can be respectively fabricated into various individual integrated circuit modules, or the plurality of the modules or steps can be fabricated into one individual integrated circuit module. Therefore, the present application is not limited to any particular hardware, software or combination thereo f.

The foregoing is only preferred embodiments of the present application, and it is not intended to limit the present application. Moreover, it will be apparent to those skilled in the art that various modifications and variations can be made to the present application. Thus, any modifications, equivalent substitutions, improvements etc. within the spirit and principle of the present application should be included within the scope of protection of the application.

## Claims

1. A method for editing an organic chemical structure formula comprising:
monitoring an input from a keyboard;
determining a focus at the organic chemical structure formal in an interactive chemical typesetting interface; and
prompting, in response to the input, executable operations for the determined focus in the interactive chemical typesetting interface.

2. The method according to claim 1, wherein when it is determined that the determined focus is an atom and the input is from a first key or a first key combination, the step of prompting comprises:
prompting that the first key or key combination together with a specific key is capable of constructing a bond connected to the atom.

3. The method according to claim 2, wherein, if the input is from a second key or a second key combination and one bond is connected with the atom, the step of prompting comprises:
prompting that the second key or key combination together with a specific key is capable of rotating the bond around the atom,
wherein, if the input is from a second key or a second key combination and a plurality of bonds are connected with the atom, the step of prompting comprises:
prompting that the second key or key combination together with a specific key is capable of moving the atom and then changing the plurality of bonds accordingly.

4. The method according to claim 2, wherein, when it is determined that the focus is a bond and the input is from a first key or a first key combination, the step of prompting comprises:
prompting that the first key or key combination together with the specific keys is capable of switching a type of the bond.

5. The method according to claim 1, wherein different operations are prompted in different colors.

6. An apparatus for editing an organic chemical structure formal comprising:
a monitoring module configured to monitor an input from a keyboard;
a focus module configured to determine a focus at the organic chemical structure formal in an interactive chemical typesetting interface; and
a prompting module configured to, in response to the input, prompt executable operations for the determined focus in the interactive chemical typesetting interface.

7. The apparatus according to claim 6, when it is determined that the determined focus is an atom and if the input is from a first key or a first key combination, the operation of prompting comprises:
prompting that the first key or key combination together with a specific key is capable of constructing a bond connected to the atom.

8. The apparatus according to claim 7, if the input is from a second key or a second key combination and one bond is connected with the atom, the operation of prompting comprises:
prompting that the second key or key combination together with a specific key is capable of rotating the bond around the atom,
wherein, if the input is from a second key and a second key combination and a plurality of bonds are connected with the atom, the operation of prompting comprises:
prompting that the second key or the key combination together with a specific key is capable of moving the atom and then changing the plurality of bonds accordingly.

9. The apparatus according to claim 7, wherein, when it is determined that the focus is a bond and the input is from a first key or a first key combination, the operation of prompting comprises:
prompting that the first key or the first key combination together with the specific keys is capable of switching a type of the bond.

10. The apparatus according to claim 6, wherein different operations are prompted in different colors.
